(19) Europäisches Patentamt
European Patent Office
Office européen des brevets

(11) Veröffentlichungsnummer: **0 085 170**
**A2**

# (12) EUROPÄISCHE PATENTANMELDUNG

(21) Anmeldenummer: 82111688.6

(22) Anmeldetag: 16.12.82

(51) Int. Cl.³: **C 07 D 229/00**
C 07 D 487/08, A 01 N 43/90
C 06 C 9/00
//(C07D487/08, 229/00, 209/00),
(C07D487/08, 249/00, 229/00)

(30) Priorität: 16.01.82 DE 3201190
06.03.82 DE 3208091
06.03.82 DE 3208090

(43) Veröffentlichungstag der Anmeldung:
10.08.83 Patentblatt 83/32

(84) Benannte Vertragsstaaten:
AT BE CH DE FR GB IT LI NL

(71) Anmelder: **BASF Aktiengesellschaft**
**Carl-Bosch-Strasse 38**
**D-6700 Ludwigshafen(DE)**

(72) Erfinder: **Rieber, Norbert, Dr.**
**Liebfrauenstrasse 1c**
**D-6800 Mannheim 51(DE)**

(72) Erfinder: **Platz, Rolf, Dr.**
**Hansastrasse 5**
**D-6800 Mannheim 1(DE)**

(72) Erfinder: **Jung, Johann, Dr., Dipl.-Landwirt**
**Hardenburgstrasse 19**
**D-6703 Limburgerhof(DE)**

(54) **Norbornanverbindungen und diese enthaltende Mittel zur Regulierung des Pflanzenwachstums sowie deren Vorprodukte.**

(57) Norbornanverbindungen der Formel

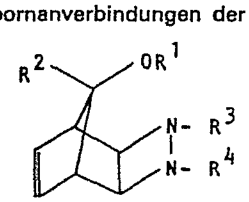

sowie deren Vorprodukte, nämlich Diazatricyclononenverbindungen der Formel

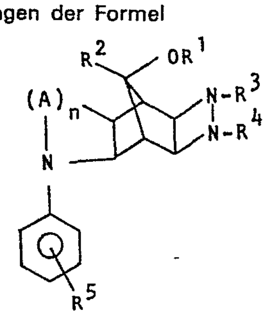

wobie $R^1$ bis $R^5$, A und n die in den Ansprüchen und der Beschreibung näher bezeichnete Bedeutung haben, deren Herstellung und Verwendung als Pflanzenschutzmittel bzw. Mittel zur Regulierung des Pflanzenwachstums.

EP 0 085 170 A2

Norbornanverbindungen und diese enthaltende Mittel zur Regulierung des Pflanzenwachstums sowie deren Vorprodukte

Die vorliegende Erfindung betrifft neue tetracyclische stickstoffhaltige Norbornanderivate und diese enthaltende Mittel zur Regulierung des Pflanzenwachstums sowie deren neue Vorprodukte.

Es ist bekannt, polycyclische stickstoffhaltige Norbornanverbindungen, z.B. das 5-(4-Chlor(oder 4-Brom)phenyl)-3,4,5,9,10-pentaazatetracyclo-[$5,4,1,0^{2,6},0^{8,11}$]-dodeca-3,9-dien zur Regulierung des Pflanzenwachstums zu verwenden (DE-OS 26 15 878, DE-OS 27 42 034).

Literaturbekannt sind auch Verbindungen des Typs

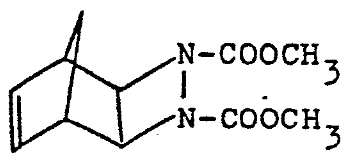

(vgl. J.Amer.Chem.Soc. 91, 5668, (1969)) und ihre Verwendung als Ausgangsprodukte zur Herstellung von Pflanzenschutzmitteln (DE-OS 21 65 878 und DE-OS 27 42 034).

Es wurde gefunden, daß die neuen Verbindungen der Formel II

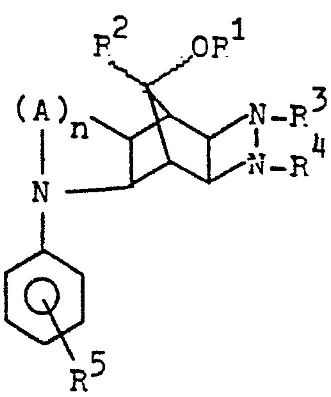 II,

Mu/HB

in der

A den Rest -N=N-

n 0 und 1

$R^1$ Wasserstoff, einen acyclischen oder cyclischen Alkyl- oder Alkylcarbonylrest (z.B. mit 1 bis 5 C-Atomen im Alkylrest) oder einen Trimethylsilylrest oder Trifluoracetylrest,

$R^2$ Wasserstoff bedeutet oder $R^1$ und $R^2$ gemeinsam eine Bindung bedeuten,

$R^3$ und $R^4$ gleich oder verschieden sind und Wasserstoff oder einen Alkoxicarbonylrest (z.B. mit 1 bis 4 C-Atomen) oder gemeinsam eine Bindung bedeuten und

$R^5$ Wasserstoff, Halogen oder Halogenalkyl (z.B. mit 1 bis 4 C-Atomen) bedeuten,

wobei die räumliche Position der Substituenten $OR^1$ und $R^2$ an der $C_1$-Brücke nicht festgelegt ist,

zur Regulierung des Pflanzenwachstums gut geeignet sind.

Die neuen Verbindungen lassen sich nach den in den DE-OS 26 15 878, 27 42 034 und 30 01 580 allgemein beschriebenen Verfahren herstellen; geeignet sind beispielsweise

1. Umsetzung der Verbindungen I, wobei $R^1$-$R^4$ die obengenannten Bedeutungen haben, mit Arylaziden ($R^5$ wie oben beschrieben) zu den Triazolin-Derivaten (n = 1).

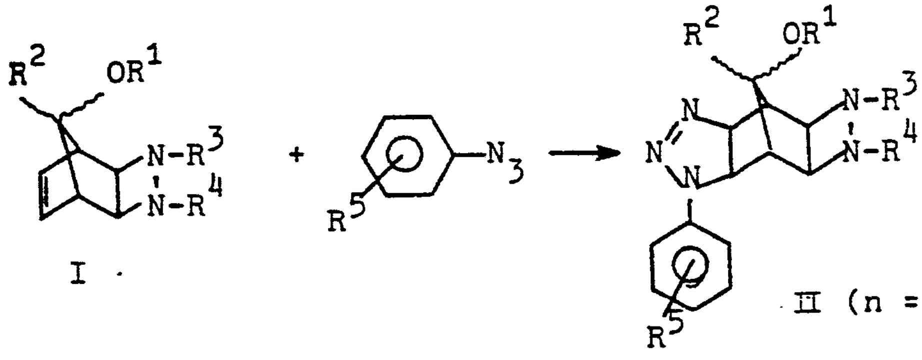

I

II (n = 1)

2. Abspaltung von $N_2$ aus den so erhaltenen Triazolin-Derivaten in Anwesenheit von Säuren zu den entsprechenden Aziridin-Derivaten (n = 0).

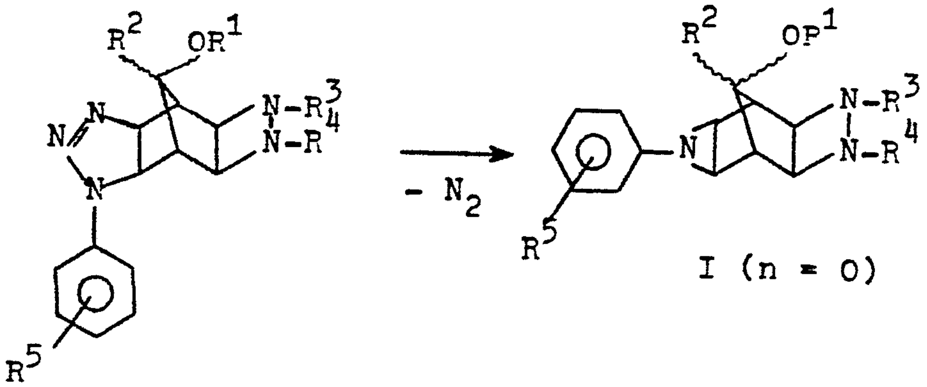


I (n = 0)

3. Verseifung und Decarboxylierung der Triazolin- bzw. Aziridin-Derivate, ($R^3$ und $R^4$ = -COOAlkyl) mit wäßriger oder alkoholischer Alkalilauge zu den entsprechenden Hydrazin-Derivaten ($R^3$ und $R^4$ = H) und Oxydation der Hydrazin-Derivate, gegebenenfalls ohne vorherige Isolierung, mit wäßriger NaOCl- oder $H_2O_2$-Lösung zu den Azoverbindungen ($R^3$ und $R^4$ bilden eine Bindung).

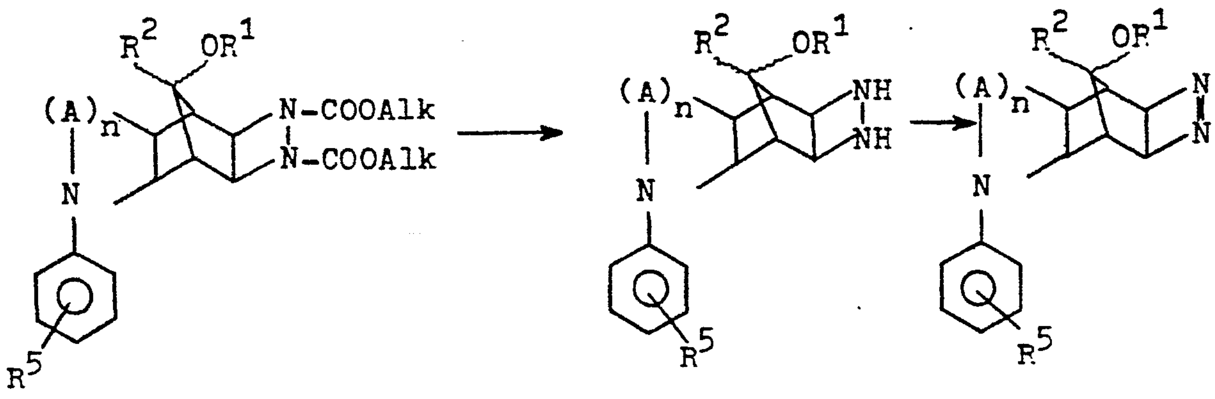

Bei der entsprechenden Umsetzung von Ester-Verbindungen der Formel II (n = 0 oder 1, $R^1$ = Alkylcarboxi, $R^2$ = H, $R^3$ und $R^4$ = -COOAlk) entstehen dabei die entsprechenden Hydrazin- bzw. Azo-Derivate mit $R^1$ und $R^2$ = H.

Die Umsetzung der Verbindungen I mit Arylaziden zu den Triazolin-Derivaten II (n = 1) wird beispielsweise mit stöchiometrischen oder nichtstöchiometrischen Mengen der Ausgangsprodukte, mit oder ohne Lösungsmittel, bei einer Temperatur von bis zu 140°C, vorzugsweise 20 bis 100°C durchgeführt. Die Wahl des Lösungsmittels richtet sich dabei nach der Löslichkeit der Ausgangskomponenten. Die Reaktionsprodukte fallen entweder beim Abkühlen bzw. bei Verwendung von polaren Solventien durch Zusatz von unpolaren Solventien aus oder lassen sich durch Einengen der Lösung gewinnen.

Die Stickstoff-Abspaltung aus den Triazolin-Derivaten zu den Aziridin-Derivaten kann beispielsweise in einem Lösungsmittel durch Zusatz von Säuren, bewirkt werden. Meist genügen katalytische Mengen von z.B. Schwefelsäure, Essigsäure oder Trifluoressigsäure, bei Temperaturen von bis zu 120°C, vorzugsweise 20 bis 80°C.

Die Verseifung und Decarboxylierung der Triazolin- oder Aziridin-Derivate II (n = 0 oder 1, $R^3$ und $R^4$ = -COOAlk) wird beispielsweise so durchgeführt, daß man die Ausgangsverbindungen mit wäßriger oder alkoholischer ($C_1$-$C_4$-Alkohol, vorzugsweise Methanol) Alkalilauge (NaOH oder KOH) bei bis zu 120°C, vorzugsweise 40 bis 80°C verseift und decarboxyliert. Die dabei entstehenden Hydrazin-Derivate II (n = 0 oder 1, $R^3$ und $R^4$ = H) können durch Absaugen und Waschen mit Wasser oder Extraktion aus der Reaktionslösung gegebenenfalls nach Zugabe von Wasser oder aus dem Rückstand nach Einengen des Reaktionsgemisches erhalten werden.

Die Hydrazin-Derivate lassen sich in wäßriger oder alkoholischer Suspension bzw. Lösung oder ohne vorherige Isolierung durch Zugabe von wäßriger NaOCl- oder $H_2O_2$-Lösung

zum Reaktionsgemisch bei bis zu 100°C, vorzugsweise 40 bis 90°C, zu den Azoverbindungen II (n = 0 oder 1, $R^3$ und $R^4$ bilden eine Bindung) oxydieren. Die Abtrennung der Endprodukte aus der Reaktionsmischung kann in entsprechender Weise wie bei den Hydrazin-Derivaten beschrieben erfolgen.

Für die Umsetzung der Verbindung II mit der Lösung von Alkalihydroxyd werden beispielsweise, abhängig von der Löslichkeit des Alkalihydroxyds, Lösungen von Alkalihydroxyd in Wasser oder Methanol in Konzentrationen von 1 bis 600 g Alkalihydroxyd in einem Liter der Lösung verwendet, wobei je Mol der Verbindung II 4 bis 10 Mole Alkalihydroxyd verwendet werden.

Für die Oxydation werden wäßrige Lösungen von Natriumhypochlorit oder Wasserstoffsuperoxyd verwendet, die je Liter Lösung 1 bis 600 g des Oxydationsmittels enthalten. Für die Oxydation werden je mol der Verbindung II 1 bis 10 mol, vorzugsweise 1 bis 2 mol, des Oxydationsmittels verwendet.

Die Substituenten der $C_1$-Brücke können in syn- oder anti--Position zum Diazavierring stehen. Die Erfindung umfaßt daher sowohl die reinen Isomeren oder als auch die Isomerengemische.

Eine Gruppe des neuen Diazatricyclononenderivate I wird vorteilhaft erhalten, wenn man Quadricyclanderivate der Formel III

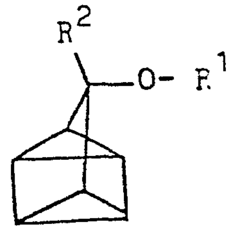

III

in der der Rest $R^1$ die vorgenannte Bedeutung hat, mit Azodicarbonsäuredialkylester der Formel IV

$$\begin{array}{l} N\text{-}COO\ Alk \\ \| \\ N\text{-}COO\ Alk \end{array} \qquad IV$$

in einem Temperaturbereich von bis zu 160°C umsetzt.

Die Alkylreste in IV können beispielsweise eine Methyl-, Ethyl-, n-Propyl-, i-Propyl-, n-Butyl-, t-Butyl-, Neopentyl-, Hexyl-, 2-Ethylhexyl-, Nonyl- oder Decylgruppe sein.

Im einzelnen können die erfindungsgemäßen Vorprodukte wie folgt erhalten werden:

Die Umsetzung der beschriebenen Quadricyclanderivate III (J.Amer. Chem.Soc. 90, 7276 (1968) und 96, 6400 (1974) mit literaturbekannten Azodicarbonsäuredialkylestern IV (Houben-Weyl 10/2, 807ff) zu Diazatricyclononenderivaten I kann mit oder ohne Lösungsmittel in einem Temperaturbereich von i.a. bis 160°C, vorzugsweise 20 bis 130°C vorgenommen werden.

Zur besseren Abführung der Reaktionswärme arbeitet man zweckmäßigerweise mit einem Lösungsmittel, z.B. Toluol, Xylol, Chlorbenzol, Petrolether, Ether, Dichlormethan, Chloroform, Methanol, Ethanol oder Isobutanol oder Gemischen solcher Lösungsmittel.

Bei Bedarf kann man die Umsetzung auch unter erhöhtem oder vermindertem Druck durchführen. Die Reaktionsteilnehmer können jeweils in stöchiometrischer Menge, im Unter- oder Überschuß eingesetzt werden.

Die Isolierung der Umsetzungsprodukte erfolgt nach allgemein üblichen Methoden, z.B. durch teilweises oder vollständiges Abdestillieren des Lösungsmittels.

Die Reinigung der Produkte kann durch Extrahieren bzw. Digerieren mit Lösungsmitteln, Umkristallisieren oder Destillation im Ölpumpenvakuum vorgenommen werden.

Die $-O-R^1$-Gruppe kann in syn- oder anti-Position zum Diaza-Vierring stehen. Daher können diese Verbindungen als reine Isomere oder als Gemisch der Isomeren, wie sie in wechselnder Zusammensetzung bei der Hestellung anfallen, vorliegen.

Die andere Gruppe von Vorprodukten I, nämlich die entsprechenden Azoverbindungen, kann aus der vorstehend beschriebenen Gruppe im Wege der Decarboxylierung und Oxidation, wie weiter oben erläutert, erhalten werden.

Die Herstellung der Ausgangsprodukte kann beispielsweise nach folgenden Vorschriften erfolgen:

Vorschrift 1

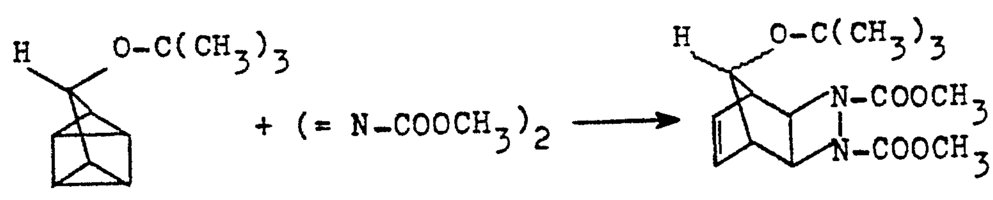

Zu 15 Teilen (Gew.-Teile) 7-t-Butoxiquadricyclan und 50 Teilen Toluol tropfte man unter Rühren bei 80°C 13 Teile Azodicarbonsäuredimethylester und rührte 12 Stunden bei 80°C. Nach Einengen des Reaktionsgemisches im Rotationsverdampfer bei 20 bis 50°C und 20 mbar wurde der Rückstand 10 Stunden mit 200 Teilen Petrolether extrahiert.

Aus der Petroletherphase erhält man durch Einengen und Trocknen des Rückstands bei 20 mbar 26 Teile (93 Mol%) 3,4-Dimethoxicarbonyl-9-t-butoxi-3,4-diaza-tricyclo-[4.2.1.$0^{2.5}$]-nona-7-en, Fp. 86°C (Toluol/Ligroin).

Entsprechend wurden hergestellt:

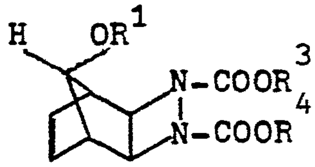

| $R^1$ | $R^3$, $R^4$ | Fp [°C] |
|---|---|---|
| $(CH_3)_3C-$ | $C_2H_5-$ | 33 |
| $CH_3CO-$ | $CH_3-$ | 176 |
| $CH_3CO-$ | $C_2H_5-$ | 57 |
| $CH_3CO-$ | $C_4H_9-$ | 29 |

<u>Vorschrift 2</u>

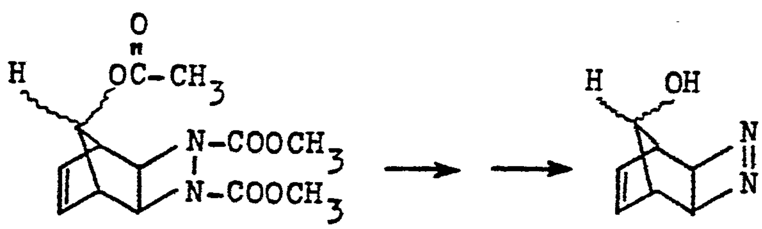

92 Teile 3.4-Dimethoxicarbonyl-9-acetoxi-3.4-diazatricyclo-[4.2.1.$0^{2.5}$]-nona-7-en und 570 Teile 20 proz. wäßrige NaOH wurden 3 Stunden bei 70°C gerührt und danach bei 40°C 356 Teile 13 proz. (Gew.-%) wäßrige NaOCl zugetropft und 1 Stunde bei 40°C nachgerührt. Das Reaktionsgemisch wurde 12 Stunden mit 300 Teilen $CH_2Cl_2$ kontinuierlich extrahiert, die organische Phase mit $MgSO_4$ getrocknet, mit Aktivkohle behandelt, eingeengt und der Rückstand mit 200 Teilen Petrolether digeriert.

Man erhielt 36.4 Teile (86 Mol%) 9-Hydroxi-3.4-diaza-tricyclo-[4.2.1.$0^{2.5}$]-nona-3.7-dien, Fp. 128°C (aus Cyclohexan).

Vorschrift 3

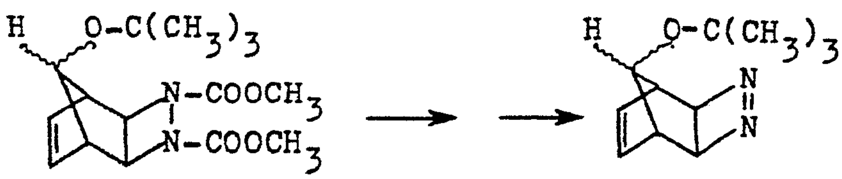

50 Teile 3.4-Dimethoxicarbonyl-9-t-butoxi-3.4-diazatricyclo-[4.2.1.$0^{2.5}$]-nona-7-en, 50 Teile KOH und 250 Teile Methanol wurden 7 Stunden am Rückfluß erhitzt. Anschliessend wurden bei 60°C 36 Teile 30 proz. wäßriges $H_2O_2$ zugetropft und noch 1 Stunde bei 60°C gerührt. Nach dem Abkühlen auf 20°C wurde das Festprodukt abgesaugt, mit 50 Teilen Methanol gewaschen und die vereinigten Filtrate im Rotationsverdampfer bei 20 bis 40°C und 20 mbar eingeengt. Der Rückstand wurde 12 Stunden im Soxhlet mit 300 Teilen Petrolether extrahiert.

Durch Einengen des Petrolethers erhielt man 28.5 Teile (93 Mol%) 9-t-Butoxi-3.4-diazatricyclo-[4.2.1.$0^{2.5}$]--nona-3.7-dien, Fp. 78°C (aus Ligroin).

Vorschrift 4

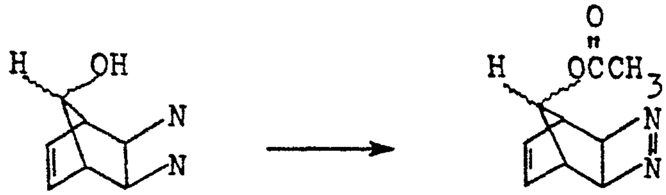

5 Teile 9-Hydroxi-3.4-diazatricyclo-[$4.2.1.0^{2.5}$]-nona-3.7-dien, 5 Teile Essigsäureanhydrid, 1 Teil Triethylamin und 100 Teile Ether wurden 24 Stunden am Rückfluß erhitzt. Anschließend wurde das Reaktionsgemisch 2 x mit 50 Teilen Wasser extrahiert, die organische Phase mit $MgSO_4$ getrocknet und im Rotationsverdampfer eingeengt.

Man erhielt 6.2 Teile (95 Mol%) 9-Acetoxi-3.4-diazatricyclo-[$4.2.1.0^{2.5}$]-nona-3.7-dien, Fp. 58°C.

Entsprechend wurden hergestellt:

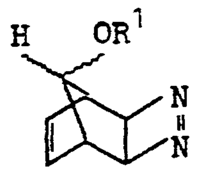

| $R^1$ |
|---|
| $CF_3CO-$ |
| $C_2H_5CO-$ |
| $C_3H_7CO-$ |
| $i-C_3H_7CO-$ |
| $C_4H_9CO-$ |
| $(CH_3)_3Si-$ |

Vorschrift 5

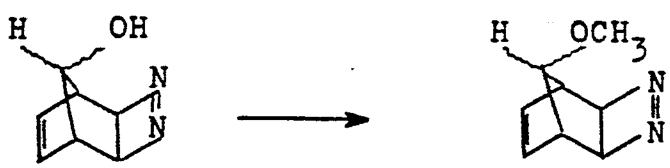

Zu 5 Teilen 9-Hydroxi-3.4-diazatricyclo-[$4.2.1.0^{2.5}$]-nona-3.7-dien und 100 Teilen Acetonitril gab man portionsweise bei 20°C unter Rühren 1 Teil NaH. Anschließend fügte man 20 Teile Methyliodid zu und rührte 12 Stunden

bei 20°C nach. Das Reaktionsgemisch wurde im Rotationsverdampfer eingeengt (20 bis 50°C, 20 mbar) und der Rückstand mit 200 Teilen Petrolether extrahiert.

Durch Einengen des Petrolethers erhielt man 5 Teile (90 Mol%) 9-Methoxi-3.4-diazatricyclo-[$4.2.1.0^{2.5}$]-nona-3.7-dien als Öl, $^1$H-NMR ($\delta$ in ppm): 6.2 (bs, 2H), 4.1 (bs, 2H), 3.1-3.3 (m, 6H).

Entsprechend wurden hergestellt:

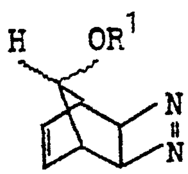

| $R^1$ |
|---|
| $C_2H_5-$ |
| $C_3H_7-$ |
| i-$C_3H_7-$ |
| $C_4H_9-$ |

Vorschrift 6

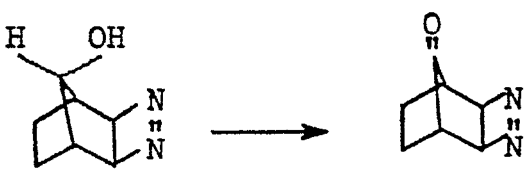

I II

Zu einer Mischung von 18,5 Teilen I, 78 Teilen Dicyclohexylcarbodiimid und 140 Teilen wasserfreies DMSO gab man unter Rühren und Kühlen bei 20 bis 30°C portionsweise 1,3 Teile Orthophosphorsäure und rührte 5 Stunden bei ca. 25°C. Nach Zugabe von 460 Teilen $CH_2Cl_2$ wurde filtriert und das Filtrat 3 Stunden bei 25°C mit 400 Teilen gesättigter wäßriger $NaHCO_3$-Lösung und 10 Teilen Soda

gerührt. Die organische Phase wurde abgetrennt, die wäßrige Phase 5 x mit 200 Teilen $CH_2Cl_2$ extrahiert und die vereinigten organischen Phasen 2 x mit 300 Teilen Wasser gewaschen. Nach Trocknen mit $MgSO_4$ und Behandeln mit Aktivkohle wurde die $CH_2Cl_2$-Phase eingeengt und der Rückstand 12 Stunden mit 200 Teilen Petrolether kontinuierlich extrahiert.

Aus der Petroletherphase erhielt man durch Einengen 14,5 Teile (79 Mol%) II, Fp. 90°C.

Die Herstellung der neuen Verbindungen der Formel I kann beispielsweise entsprechend folgenden Beispielen erfolgen:

Beispiel 1

A. 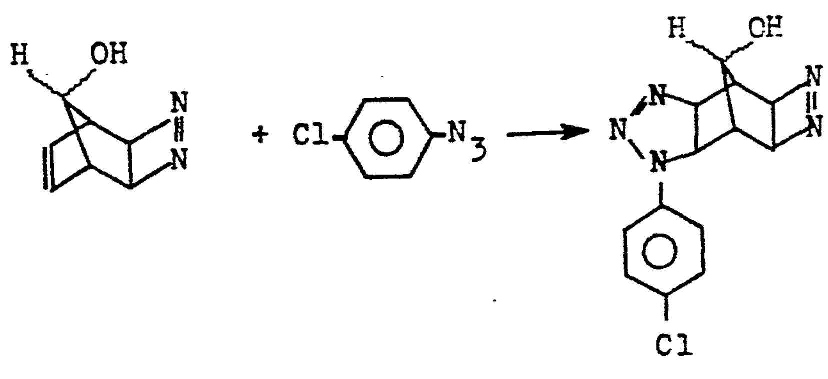

30 Teile 9-Hydroxi-3.4-diazatricyclo-[$4.2.1.0^{2.5}$]-nona-3.7-dien und 34 Teile p-Chlorphenylazid wurden in 100 Teilen Toluol 3 Stunden unter Rühren auf 80°C erhitzt. Nach dem Einengen im Rotationsverdampfer bei 20 bis 40°C und 20 mbar wurde der Rückstand nacheinander mit 100 Teilen Diethylether und 100 Teilen Petrolether digeriert und bei 20°C und 1 mbar getrocknet.

Man erhielt 59 Teile (92 Mol%) 8-(4-Chlorphenyl)-12-hydroxi-3.4.8.9.10-pentazatetracyclo-[$5.4.1.0^{2.5}.0^{7.11}$]-dodeca-3.9-dien, Fp. 178°C (Zers.) (Verbindung Nr. 1).

B. 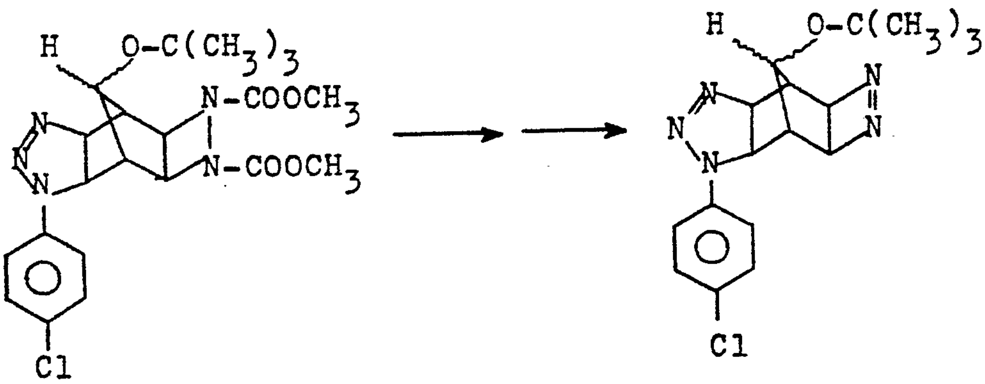

400 Teile 3.4-Dimethoxicarbonyl-8-(4-Chlorphenyl)-12-t-butoxi-3.4.8.9.10-pentazatetracyclo-[$5.4.1.0^{2.5}.0^{7.11}$]-dodeca-9-en (Verbindung Nr. 9) wurden in 1000 Teilen 25 proz. wäßriger Natronlauge suspendiert und 6 Stunden bei 85°C gerührt. Anschließend wurden bei 60°C 1150 Teile 13 proz. wäßrige NaOCl in 2 Stunden zugegeben und 4 Stunden bei 60°C nachgerührt. Nach Abkühlen auf 20°C wurde das Festprodukt abgesaugt, mit 3000 Teilen Wasser gewaschen und bei 30°C und 1 mbar getrocknet.

Man erhielt 270 Teile (96 Mol%) 8-(4-Chlorphenyl)-12-t-butoxi-3.4.8.9.10-pentazatetracyclo-[$5.4.1.0^{2.5}.0^{7.11}$]-dodeca-3.9-dien, Fp. 178°C (Zers.). (Verbindung Nr. 2).

C. 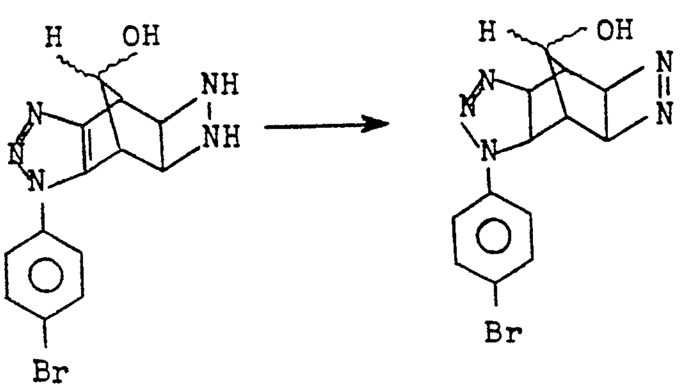

Zu 50 Teilen 8-(4-Bromphenyl)-12-hydroxi-3.4.8.9.10-pentaazatetracyclo-[5.4.1.$0^{2.5}$.$0^{7.11}$]-dodeca-9-en (Verbindung Nr. 69) in 400 Teilen Methanol und 5 Teilen 25 gew.proz. wäßrige NaOH tropfte man unter Rühren bei 65°C innerhalb von 1 Stunde 35 Teile 30 proz. wäßrige $H_2O_2$ zu und rührte 5 Stunden bei 65°C nach. Nach dem Abkühlen auf 20°C fügte man 1500 Teile Wasser zu, saugte ab, wusch den Rückstand mit 300 Teilen Wasser und trocknete bei 30°C und 1 mbar.

Man erhielt 44 Teile (88 Mol%) 8-(4-Bromphenyl)-12-hydroxi-3.4.8.9.10-pentaza-tetracyclo-[5.4.1.$0^{2.5}$.$0^{7.11}$]-dodeca-3.9-dien, Fp. 187°C (Zers.) (Verbindung Nr. 3).

Entsprechend wurden folgende Verbindungen hergestellt:

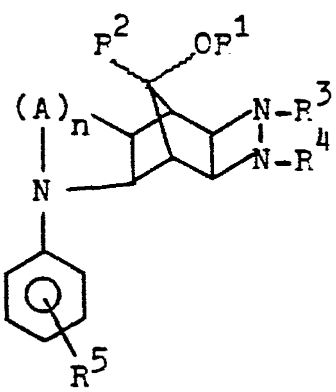

A : -N=N-

Für den Fall, daß $R^1$ und $R^2$ eine Bindung bedeuten, ergibt sich in 12-Stellung des Moleküls folgender Rest

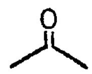

| Verbindung Nr. | $R^1$ | $R^2$ | $R^3$ | $R^4$ | $R^5$ | n | Fp [°C] |
|---|---|---|---|---|---|---|---|
| 4 | H- | H- | eine Bindung | | H- | 1 | 148 (Zers.) |
| 5 | " | " | " | " | m-$CF_3$- | 1 | 177 " |
| 6 | " | " | " | " | p-F- | 1 | 181 " |
| 7 | " | " | " | " | o-Br- | 1 | 166 " |
| 8 | " | " | " | " | m-$CF_3$- | 0 | 135 " |
| 9 | $(CH_3)_3C$- | " | $CH_3OCO$- | $CH_3OCO$- | o-Cl- | 1 | 186 " |
| 10 | " | " | " | " | m-$CF_3$- | 1 | 183 " |
| 11 | " | " | " | " | p-Br- | 1 | 210 " |
| 12 | " | " | $C_2H_5OCO$- | $C_2H_5OCO$- | H- | 1 | 184 " |
| 13 | " | " | " | " | p-Br- | 1 | 179 " |
| 14 | " | " | eine Bindung | | m-$CF_3$- | 1 | 192 " |
| 15 | $(CH_3)_3C$- | H- | eine Bindung | | p-Br- | 1 | 200 (Zers.) |
| 16 | " | " | " | " | p-Br- | 0 | 133 " |
| 17 | eine Bindung | | " | " | m-$CF_3$- | 1 | 168 " |
| 18 | " " | | " | " | p-Cl- | 1 | 186 " |
| 19 | " " | | " | " | m-Cl- | 1 | 173 " |
| 20 | " " | | " | " | p-Br- | 1 | 187 " |
| 21 | " " | | " | " | m-$CF_3$- | 0 | 138 " |
| 22 | " " | | " | " | p-Cl- | 0 | 151 " |
| 23 | $CF_3CO$- | H- | " | " | p-Cl- | 1 | 180 " |
| 24 | " | " | " | " | m-$CF_3$- | 1 | 177 " |
| 25 | $CH_3CO$- | " | $CH_3OCO$- | $CH_3OCO$- | p-Cl- | 1 | 197 " |
| 26 | " | " | " | " | o-Cl- | 1 | 174 " |
| 27 | " | " | " | " | m-$CF_3$- | 1 | 175 " |
| 28 | " | " | " | " | p-Br- | 1 | 153 " |
| 29 | " | " | $C_4H_9OCO$- | $C_4H_9OCO$- | H- | 1 | 191 " |
| 30 | " | " | " | " | p-Cl- | 1 | 182 " |
| 31 | " | " | " | " | p-F- | 1 | 180 " |

| Verbindung Nr. | $R^1$ | $R^2$ | $R^3$ | $R^4$ | $R^5$ | n | Fp [°C] |
|---|---|---|---|---|---|---|---|
| 32 | $CH_3CO$ | H | eine | Bindung | p-Cl- | 1 | 185 " |
| 33 | " | " | " | " | m-$CF_3$- | 1 | 152 " |
| 34 | " | " | " | " | m-Br- | 1 | 187 " |
| 35 | " | " | " | " | p-Br- | 1 | 193 " |
| 36 | $CH_3$- | " | " | " | p-Br- | 0 | 139 " |
| 37 | " | " | " | " | m-$CF_3$- | 1 | 176 " |
| 38 | " | " | " | " | p-Br- | 1 | 191 " |
| 39 | " | " | " | " | p-Cl- | 1 | 183 " |
| 40 | " | " | " | " | p-Cl- | 0 | 138 " |
| 41 | $C_2H_5$- | " | " | " | H- | 1 | 178 " |
| 42 | " | " | " | " | p-Cl- | 1 | 179 " |
| 43 | " | " | " | " | p-F- | 1 | 174 " |
| 44 | " | " | " | " | p-Br- | 0 | 133 " |
| 45 | $C_2H_5CO$- | " | " | " | p-Cl- | 1 | 189 " |
| 46 | " | " | " | " | m-$CF_3$- | 1 | 185 " |
| 47 | $CH_3CH(CH_3)CO$- | H- | eine | Bindung | p-Br- | 1 | 149 (Zers.) |
| 77 | $(CH_3)_3Si$- | " | " | " | p-Cl- | 1 | 186 " |
| 78 | " | " | " | " | m-$CF_3$- | 1 | 189 " |
| 79 | " | " | " | " | p-Br- | 1 | 178 " |

Beispiel 2

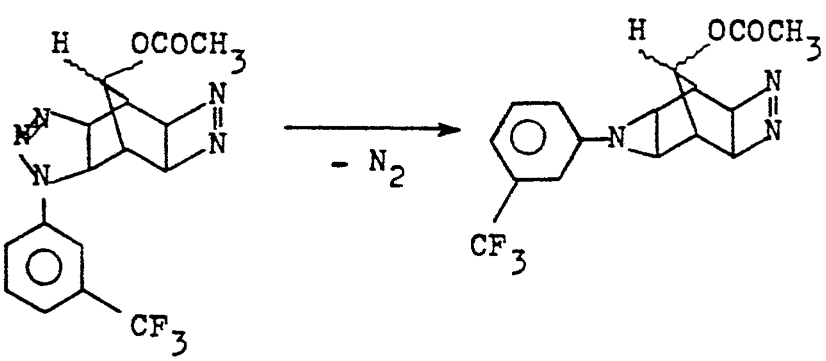

10 Teile 8-(3-Trifluormethyl-phenyl)-12-acetoxi-3.4.8.9.10-pentaza-tetracyclo-[$5.4.1.0^{2.5}.0^{7.11}$]-dodeca-3.9-dien (Verbindung Nr. 33) wurden in 50 Teilen Essigsäure und 150 Teilen Ether 5 Stunden bei 25°C gerührt. Nach dem Einengen im Rotationsverdampfer bei 20°C und 1 bis 20 mbar wurde der Rückstand nacheinander mit 50 Teilen Ether und Petrolether digeriert.

Nach dem Trocknen bei 1 mbar erhielt man 9.1 Teile (94 Mol%) 8-(3-Trifluormethyl-phenyl)-12-acetoxi-3.4.8-triazatetracyclo-[$4.3.1.0^{2.5}.0^{7.9}$]-deca-3-en, Fp. 154°C (Zers.) (Verbindung Nr. 48).

Entsprechend wurden folgende Verbindungen hergestellt:

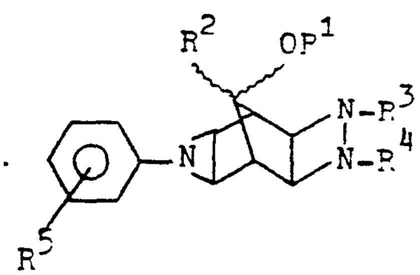

| Verbindung Nr. | $R^1$ | $R^2$ | $R^3$ | $R^4$ | $R^5$ | Fp [°C] |
|---|---|---|---|---|---|---|
| 49 | H- | H- | eine Bindung | | H- | 132 (Zers.) |
| 50 | " | " | " | " | p-Cl- | 124 " |
| 51 | " | " | " | " | p-Br- | 141 " |
| 52 | $(CH_3)_3C-$ | " | $CH_3OCO-$ | $CH_3OCO-$ | m-$CF_3$- | 98 |
| 53 | " | " | " | " | p-Cl- | 74 |
| 54 | " | " | $C_2H_5OCO-$ | $C_2H_5OCO-$ | p-Br- | 60 |
| 55 | " | " | eine Bindung | | p-Cl- | 149 (Zers.) |
| 56 | " | " | " | " | m-$CF_3$- | 121 " |
| 57 | eine Bindung | | " | " | p-Br- | 129 " |
| 58 | $CF_3CO-$ | H- | " | " | p-Cl- | 82 |

| Verbindung Nr. | $R^1$ | $R^2$ | $R^3$ | $R^4$ | $R^5$ | Fp [°C] |
|---|---|---|---|---|---|---|
| 59 | $CH_3CO-$ | H- | $CH_3OCO-$ | $CH_3OCO-$ | p-Cl- | 87 |
| 60 | " | " | " | " | $m-CF_3-$ | 61 |
| 61 | " | " | " | " | p-Br- | 64 |
| 62 | " | " | eine Bindung | | p-Cl- | 126 (Zers.) |
| 63 | $CH_3-$ | " | " | " | $m-CF_3-$ | 131 " |
| 64 | " | " | " | " | p-Br- | 144 " |
| 65 | $C_2H_5-$ | " | " | " | $m-CF_3-$ | 128 " |
| 80 | $(CH_3)_3Si-$ | " | " | " | p-Cl- | 150 " |
| 81 | " | " | " | " | $m-CF_3-$ | 128 " |

Beispiel 3

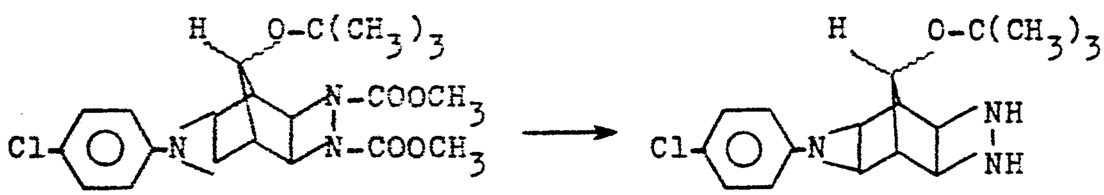

300 Teile 3.4-Dimethoxicarbonyl-8-(4-chlorphenyl)-12-t-butoxi-3.4.8-triaza-tetracyclo-[$4.3.1.0^{2.5}.0^{7.9}$]-decan (Verbindung Nr. 53), 150 Teile NaOH und 2000 Teile Methanol erhitzte man unter Rühren 6 Stunden auf 65°C. Danach saugte man ab, wusch den Rückstand mit 500 Teilen Methanol und engte die Filtrate im Rotationsverdampfer bei 20 bis 40°C und 20 mbar ein. Der Rückstand wurde mit 1000 Teilen Wasser gewaschen, bei 20 bis 40°C und 1 mbar getrocknet.

Man erhielt 185 Teile (84 Mol%) 8-(4-Chlorphenyl)-12-t--butoxi-3.4.8-triaza-tetracyclo-[4.3.1.$0^{2.5}$.$0^{7.9}$]-decan, Fp. 89°C (Verbindung Nr. 66).

Entsprechend wurden folgende Verbindungen hergestellt:

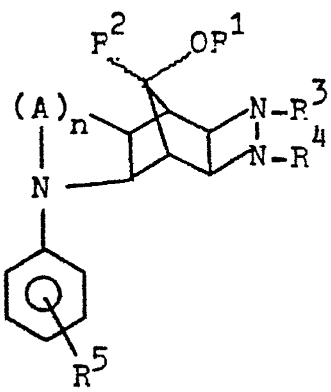

A: -N=N-
$R^3$: H
$R^4$: H

| Verbindung Nr. | $R^1$ | $R^2$ | $R^5$ | n | Fp [°C] |
|---|---|---|---|---|---|
| 67 | H- | H- | H- | 1 | 123 (Zers.) |
| 68 | H- | H- | p-Cl- | 1 | 142 " |
| 69 | H- | H- | p-Br- | 1 | 137 " |
| 70 | H- | H- | m-$CF_3$- | 1 | 150 " |
| 71 | H- | H- | p-Cl- | 0 | 83 |
| 72 | H- | H- | m-$CF_3$- | 0 | 41 |
| 73 | $(CH_3)_3C$- | H- | p-Cl- | 1 | 139 (Zers.) |
| 74 | " | H- | m-$CF_3$- | 1 | 148 " |
| 75 | " | H- | p-Br- | 1 | 160 " |
| 76 | " | H- | m-$CF_3$- | 0 | 74 |

Die neuen Wirkstoffe greifen in den Stoffwechsel der Pflanzen ein und können deshalb als Wachstumsregulatoren eingesetzt werden.

Für die Wirkungsweise von Pflanzenwachstumsregulatoren gilt nach den bisherigen Erfahrungen, daß ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von den geoklimatischen Faktoren, z.B. Sonnenscheindauer, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von der angewendeten Konzentrationen der aktiven Substanz.

In jedem Falle sollen Wachstumsregulatoren die Kulturpflanzen in gewünschter Weise positiv beeinflussen.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den neuen Verbindungen läßt sich das vegetative Wachstum der Pflanzen stark hemmen, was sich insbesonders in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachten Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Ein weiterer Mechanismus der Ertragssteigerung mit Wachstumshemmern beruht darauf, daß die Nährstoffe in stärkerem Maße der Blüten- und Fruchtbildung zugutekommen, während das vegetative Wachstum eingeschränkt wird. Ferner kann so wegen der relativ geringen Blatt- bzw. Pflanzenmasse dem Befall mit verschiedenen, insbesondere pilzlichen Krankheiten vorgebeugt werden.

Die Hemmung des vegetativen Wachstums ermöglicht außerdem bei vielen Kulturpflanzen eine dichtere Bepflanzung, so daß ein Mehrertrag, bezogen auf die Bodenfläche erzielt werden kann. Die erfindungsgemäßen Verbindungen eignen sich besonders zur Hemmung des vegetativen Wachstums bei Kulturpflanzen wie Soja, Sonnenblumen, Erdnüssen, Raps, Zierpflanzen, Baumwolle, Reis und Gräsern.

B. Mit den neuen Wirkstoffen lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt und Sprossteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z.B. Getreide wie Weizen, Gerste, Roggen, Hafer und Reis oder Mais oder Gräsern als auch insbesondere bei Dikotylen (z.B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die neuen Wirkstoffe können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,001 bis 12 kg/ha bevorzugt 0,01 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung oder in einer Lösung von Wasser unter Zusatz von mit Wasser mischbaren organischen Lösungsmitteln wie Methanol oder anderen niederen Alkoholen, Aceton, Dimethylformamid

oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Die erfindungsgemäßen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d.h. die Wirksamkeit des Kombinationsproduktes ist größer als die addierten Wirksamkeiten der Einzelkomponenten.

In den nachfolgenden Versuchen wird die Wirkung der erfindungsgemäß verwendbaren Stoffe als Pflanzenwachstumsregulatoren dargestellt, ohne die Möglichkeit weiterer Anwendungen als Wachstumsregulatoren auszuschließen.

Die folgenden Versuche erläutern die biologische Wirkung der neuen Verbindungen.

Vergleichssubstanzen:

A = 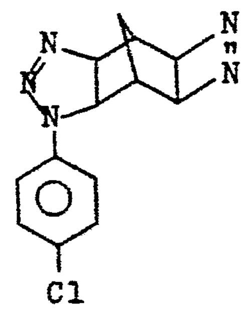

B = 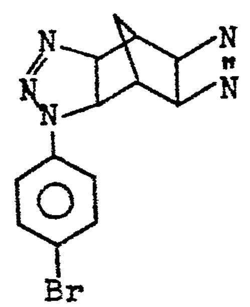

C = 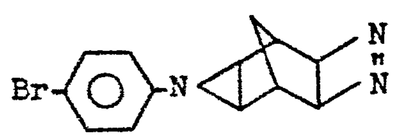

Versuch

Prüfung auf wachstumsregulierende Wirkung

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Prüfsubstanzen in wäßriger Aufbereitung am Tage der Einsaat auf das Saatbett gegossen.

Im Nachlaufverfahren wurden die zu prüfenden Substanzen in wäßriger Aufbereitung auf die Pflanzen gesprüht. Die

beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

In diesem Versuch zeigten die Wirkstoffe 39 und 77 eine wesentlich stärkere Reduzierung des Längenwachstums bei Sommergerste im Vorauflaufverfahren bei Aufwandmengen von 3 und 12 mg Wirkstoff je Gefäß als die Verbindungen A, B und C.

In einem weiteren Versuch zeigten die Wirkstoffe 1, 3, 32 und 48 eine wesentlich stärkere Reduzierung des Längenwachstums bei Rasen im Nachauflaufverfahren bei Aufwandmengen von 1,5 und 6 mg Wirkstoff je Gefäß als die Verbindungen A, B und C.

<u>Beispiel I</u>

20 Teile des Wirkstoffs 1 werden mit 12 Teilen Calciumsalz der Dodecylbenzolsulfonsäure, 8 Teilen Fettalkoholpolyglykolether, 2 Teilen Natriumsalz eines Phenolsulfonsäure-Harnstoff-Kondensats, 68 Teilen eines paraffinischen Mineralöls innig vermischt. Man erhält eine stabile ölige Dispersion.

Beispiel II

3 Gewichtsteile des Wirkstoffs 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel III

30 Gewichtsprozent des Wirkstoffs 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig ver--mischt. Man erhält auf diese Weise eine Aufbereitung mit guter Haftfähigkeit.

Beispiel IV

40 Gewichtsteile des Wirkstoffs 3 werden mit 10 Teilen Natriumsalz eines Phenolsulfonsäure-Formaldehyd-Kondensats, 2 Teilen Kieselsäuregel und 48 Teilen Wasser innig vermischt. Man erhält eine stabile wäßrige Dispersion. Durch Verdünnen mit Wasser erhält man eine wäßrige Dispersion.

Beispiel V

20 Gewichtsteile des Wirkstoffs 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in

100 000 Gewichtsteilen Wasser erhält man eine wäßrige Dispersion.

Beispiel VI

20 Gewichtsteile des Wirkstoffs 39 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

Beispiel VII

80 Gewichtsteile des Wirkstoffs 1 werden mit 3 Gewichtsteilen des Natriumsalzes der Diisobutylnaphthalin-alpha--sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

Die Wirkstoffe können auch mit gutem Erfolg im Ultra--Low-Volume-Verfahren (ULV) verwendet werden, wobei es möglich ist, Formulierungen mit mehr als 95 Gew.% Wirkstoff oder sogar den Wirkstoff ohne Zusätze auszubringen.

## Patentansprüche

1. Diazatricyclononenverbindung der Formel I

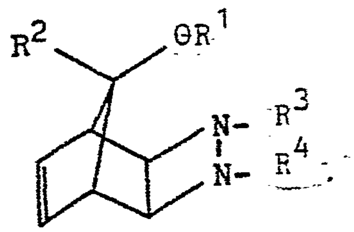

I

in der entweder

$R^1$ Wasserstoff, einen acyclischen oder cyclischen Alkyl- oder Alkylcarbonylrest, einen Trimethylsilylrest oder einen Trifluoracetylrest und

$R^2$ Wasserstoff bedeutet oder

$R^1$ und $R^2$ gemeinsam eine Bindung bedeuten, wobei die räumliche Position der Substituenten $OR^1$ und $R^2$ an der $C_1$-Brücke nicht festgelegt ist, sowie

$R^3$ und $R^4$ gleich oder verschieden sind und Wasserstoff oder einen Alkoxicarbonylrest oder gemeinsam eine Bindung bedeuten.

2. Norbornanverbindung der Formel

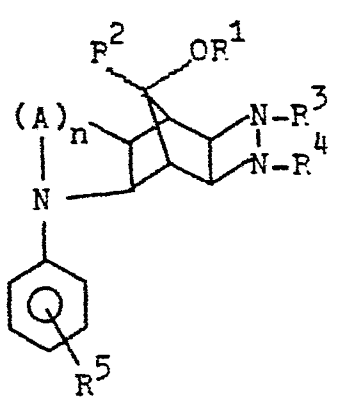

II,

in der

A den Rest -N=N-

n 0 und 1

$R^1$ bis $R^4$ die im Anspruch 1 angegebene Bedeutung haben und

$R^5$ Wasserstoff, Halogen oder Halogenalkyl bedeuten.

3. Verfahren zur Herstellung der Verbindungen gemäß Anspruch 1, dadurch gekennzeichnet, daß man entweder Quadricyclanderivate der Formel III

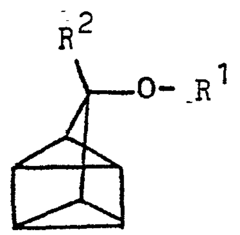

III

in der $R^1$ und $R^2$ die vorgenannte Bedeutung haben, mit Azodicarbonsäuredialkylester der Formel IV

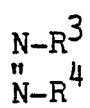

IV

in der $R^3$ und $R^4$ die vorgenannte Bedeutung haben, unterhalb von 160°C umsetzt,

oder

in einem 1. Schritt ein Quadricyclanderivat der Formel V

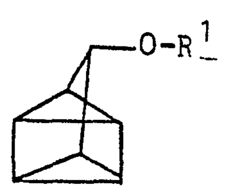

V,

in der $R^1$ die in Anspruch 1 angegebene Bedeutung hat, mit einem Azodicarbonsäuredialkylester IV unterhalb von 150°C zu einer Diazatricyclononenverbindung umsetzt und diese in einem 2. Schritt mit an sich bekannten Methoden durch Hydrolyse, Decarboxylierung und anschließender Oxidation in eine Verbindung der Formel VI

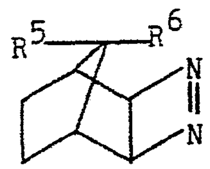 VI,

in der einer der Reste $R^5$ und $R^6$ ein Wasserstoffatom und der andere eine $OR^7$-Gruppe bedeutet (dabei steht $R^7$ für ein Wasserstoffatom, einen geradkettigen oder verzweigten Alkylrest mit 1 bis 10 Kohlenstoffatomen oder eine Trimethylsilylgruppe) überführt, woraus man, wenn $R^7$ ein Wasserstoffatom ist, gegebenenfalls in einem 3. Schritt durch Alkylierung oder Silylierung eine Verbindung der Formel VII

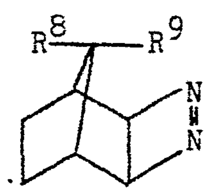 VII,

in der einer der Reste $R^8$ und $R^9$ ein Wasserstoffatom und der andere eine $O-R^1$-Gruppe bedeutet, wobei $R^1$ ein geradkettiger oder verzweigter Alkylrest mit 1 bis 10 Kohlenstoffatomen oder eine Trimethylsilylgruppe sein kann, oder durch Acylierung Verbindungen der Formel VII, in der einer der Reste $R^8$ und $R^9$ ein Wasserstoffatom und der andere ein geradkettiger oder verzweigter Alkoxyrest mit 1 bis 10 Kohlenstoffatomen

oder eine Trifluoracetoxygruppe sein kann, oder durch Oxidation die Ketoverbindung der Formel VIII

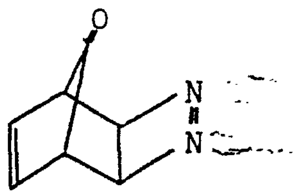

VIII

herstellt.

4. Verwendung der Verbindungen gemäß Anspruch 1 zur Herstellung der Verbindungen gemäß Anspruch 2.

5. Mittel zur Regulierung des Pflanzenwachstums, enthaltend eine Verbindung gemäß Anspruch 2.

6. Verfahren zur Regulierung des Pflanzenwachstums, dadurch gekennzeichnet, daß man die Pflanzen oder den Boden oder den Samen der Pflanzen behandelt mit einer Verbindung gemäß Anspruch 1.

7. Verwendung der Verbindung gemäß Anspruch 1, in der die Reste $R^1$ und $R^2$ zusammen ein Sauerstoffatom bedeuten, als schwermetallfreier Initialzünder.